(19)

Europäisches
Patentamt

European
Patent Office

Office européen
des brevets

(11)  **EP 3 177 933 B1**

(12)  **EUROPEAN PATENT SPECIFICATION**

(45) Date of publication and mention
of the grant of the patent:
**19.02.2020   Bulletin 2020/08**

(21) Application number: **15734617.2**

(22) Date of filing: **29.06.2015**

(51) Int Cl.:
**G01N 33/86** *(2006.01)*        **G01N 33/66** *(2006.01)*
**G01N 33/573** *(2006.01)*

(86) International application number:
**PCT/EP2015/064670**

(87) International publication number:
**WO 2016/020119 (11.02.2016 Gazette 2016/06)**

(54) **BIOMARKERS FOR PREDICTING DEGREE OF WEIGHT LOSS**

BIOMARKER ZUR VORHERSAGE DES GRADS DES GEWICHTSVERLUSTS

BIOMARQUEURS POUR PRÉDIRE LE DEGRÉ DE PERTE DE POIDS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.08.2014   EP 14179968**

(43) Date of publication of application:
**14.06.2017   Bulletin 2017/24**

(73) Proprietor: **Société des Produits Nestlé S.A.
1800 Vevey (CH)**

(72) Inventors:
• **HAGER, Jorg
25300 Houtaud (FR)**
• **IRINCHEEVA, Irina
1304 Cossonay-Ville (CH)**
• **VALSESIA, Armand
1022 Chavannes-près-Renens (CH)**
• **SARIS, Wim
6200 MD Maastricht (NL)**
• **ASTRUP, Arne
1958 Frederiksberg C (DK)**

(74) Representative: **Kamibayashi, Lynne
Société des Produits Nestlé S.A.
Avenue Nestlé 55
1800 Vevey (CH)**

(56) References cited:
**US-A1- 2011 124 121**

• **WAHL SIMONE ET AL: "Metabolomics reveals
determinants of weight loss during lifestyle
intervention in obese children",
METABOLOMICS, vol. 9, no. 6, December 2013
(2013-12), pages 1157-1167, XP002733703,**
• **DATABASE BIOSIS [Online] BIOSCIENCES
INFORMATION SERVICE, PHILADELPHIA, PA,
US; June 2007 (2007-06), SANTOSA SYLVIA ET
AL: "An investigation of hormone and lipid
associations after weight loss in women",
XP002733704, Database accession no.
PREV200700542889**
• **ELSE-MARIE BLADBJERG ET AL: "Long-term
effects on haemostatic variables of three ad
libitum diets differing in type and amount of fat
and carbohydrate: a 6-month randomised study
in obese individuals", BRITISH JOURNAL OF
NUTRITION, vol. 104, no. 12, 30 July 2010
(2010-07-30), pages 1824-1830, XP055158343,
ISSN: 0007-1145, DOI:
10.1017/S0007114510002837**
• **MENNEN LOUISE I ET AL: "Dietary effects on
coagulation factor VII vary across genotypes of
the R/Q353 polymorphism in elderly people",
JOURNAL OF NUTRITION, vol. 128, no. 5, May
1998 (1998-05), pages 870-874, XP002733705,
ISSN: 0022-3166**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF INVENTION

The present invention provides a number of biomarkers and biomarker combinations that can be used to predict the degree of weight loss attainable by applying one or more dietary interventions to a subject.

BACKGROUND

Obesity is a chronic metabolic disorder that has reached epidemic proportions in many areas of the world. Obesity is the major risk factor for serious co-morbidities such as type 2 diabetes mellitus, cardiovascular disease, dyslipidaemia and certain types of cancer (World Health Organ Tech Rep Ser. 2000;894:i-xii, 1-253).
It has long been recognized that low calorie dietary interventions can be very efficient in reducing weight and that this weight loss is generally accompanied by an improvement for the risk of obesity related co-morbidities, in particular type 2 diabetes mellitus (World Health Organ Tech Rep Ser. 2000;894:i-xii, 1-253). Empirical data suggests that a weight loss of at least 10% of the initial weight results in a considerable decrease in risk for obesity related co-morbidities (World Health Organ Tech Rep Ser. 2000;894:i-xii, 1-253). However, the capacity to lose weight shows large inter-subject variability.
Some studies (e.g. Ghosh, S. et al., Obesity (Silver Spring), (2011) 19(2):457-463) illustrate that a percentage of the population do not successfully lose weight on a low calorie diet. This leads to an unrealistic expectation of weight loss, which in turn causes non-compliance, dropouts and generally unsuccessful dietary intervention.
Some studies also demonstrate that there are methods in the art for monitoring weight loss which include monitoring levels of particular biomarkers in plasma (e.g. Lijnen et al., Thromb Res. 2012 Jan, 129(1): 74-9; Cugno et al., Intern Emerg Med. 2012 Jun, 7(3): 237-42; and Bladbjerg et al., Br J Nutr. 2010 Dec, 104(12): 1824-30). However, these methods do not provide a prediction or indication of the degree of weight loss attainable by a particular subject. There is no predictive value in looking at the correlation of biomarker levels with weight loss.
The solution for successful planning and design of dietary interventions, for example low calorie diets, lies in the availability of a method which predicts a weight loss trajectory. Such a method would be useful to assist in modifying a subject's lifestyle, e.g. by a change in diet, and also to stratify subjects into adapted treatment groups according to their biological weight loss capacity.
United States Patent Application US 2011/0124121 discloses a method for predicting weight loss success. The methods disclosed comprises selecting a patient who is undergoing or considering undergoing a weight loss therapy such as gastric banding, measuring one or more hormone responses of the patient to caloric intake and predicting success of a weight loss therapy based on the hormone response. The hormones measured are gastrointestinal hormones such as a pancreatic hormone.
European Patent Application EP 2 420 843 discloses a method for determining the probability that a person will maintain weight loss after an intentional weight loss by determining the level of angiotensin I converting enzyme (ACE) before and after the dietary period.
There is, however, still a need for a method of accurately predicting the degree of weight loss in a subject. Consequently, it was the objective of the present invention to provide biomarkers that can be detected easily and that can facilitate the prediction of weight loss in a subject. Such biomarkers can be used to predict weight trajectory of a subject prior to a dietary intervention. These biomarkers can be used to optimise dietary intervention and assist in lifestyle modifications.

SUMMARY OF THE INVENTION

The present invention investigates the level of one or more biomarkers in order to predict the degree of weight loss attainable by applying one or more dietary interventions to a subject. In particular, the inventors have found that certain biomarkers can be used to reliably predict the weight loss attainable by a subject following a low calorie diet.
Accordingly the present invention provides in one aspect a method for predicting the degree of weight loss attainable by applying one or more dietary interventions to a subject, said method comprising determining the level of fructosamine in one or more samples obtained from the subject.
In one embodiment, the method further comprises determining the level of factor VII in one or more samples.
In one embodiment, the method further comprises determining the level of adiponectin in one or more samples. The method may also comprise determining the level of insulin in one or more samples.
In one embodiment, the one or more samples are derived from blood, e.g. a blood plasma sample.
The level of the one or more biomarkers may be compared to a reference value, wherein the comparison is indicative of the predicted degree of weight loss attainable by the subject. The reference value may be based on a value (e.g. an average) of the one or more biomarkers in a population of subjects who have previously undergone the dietary intervention.

In one embodiment, a level of fructosamine is determined, and a decrease in the level of fructosamine in the sample relative to a reference value is indicative of a greater degree of weight loss in the subject. Preferably the fructosamine levels are determined by measuring glycated albumin in the one or more samples.

In another embodiment, a level of factor VII is determined, and an increase in the level of factor VII in the sample relative to a reference value is indicative of a greater degree of weight loss in a subject.

In another embodiment, a level of adiponectin is determined, and an increase in the level of adiponectin in the sample relative to a reference value is indicative of a greater degree of weight loss in a subject.

In another embodiment, a level of insulin is determined, and a decrease in the level of insulin in the sample relative to a reference value is indicative of a greater degree of weight loss in a subject.

In another embodiment, levels of each of fructosamine, factor VII, adiponectin and insulin are determined, and decreased levels of fructosamine and insulin and increased levels of factor VII and adiponectin in the sample relative to reference values is indicative of a greater degree of weight loss in a subject.

Preferably the dietary intervention is a low calorie diet. In one embodiment, the low calorie diet comprises a calorie intake of about 600 to about 1200 kcal/day. The low calorie diet may comprise administration of at least one diet product. Preferably the diet product is Optifast® or Modifast®. The low calorie diet may also comprise administration of up to, for example, about 400 g vegetables/day.

In one embodiment, the diet may comprise a product such as Optifast® or Modifast®. This may be supplemented with three portions of non-starchy vegetables such that the total energy intake is about 2.5 MJ (600 kcal/day). This may be be further supplemented with at least 2 L of water or other energy free beverages per day.

In another embodiment, the diet may comprise, for example, a composition which is 46.4% carbohydrate, 32.5% protein and 20.1% with fat, vitamins, minerals and trace elements; 2.1MJ per day (510 kcal/day); This may be supplemented with three portions of non-starchy vegetables such that the total energy intake is about 2.5 MJ (600 kcal/day). This may be be further supplemented with at least 2 L of water or other energy free beverages per day.

In one embodiment, the low calorie diet has a duration of up to 12 weeks, e.g. 6 to 12 weeks.

In one embodiment, the method further comprises combining the level of the one or more biomarkers with one or more anthropometric measures and/or lifestyle characteristics of the subject. Preferably the anthropometric measure is selected from the group consisting of gender, weight, height, age and body mass index, and the lifestyle characteristic is whether the subject is a smoker or a non-smoker.

In one embodiment, the degree of weight loss is represented by the body mass index (BMI) that a subject is predicted to attain by applying the dietary intervention. This may be termed BMI2 and be calculated using formula (1):

$$\text{BMI2} = c_1 * \text{BMI1}_i + c_2 \text{ (if subject i is female)} + c_3 * \text{age} - c_4 * \text{factor VII}_i + c_5 * \text{fructosamine}_i - c_6 * \text{adiponectin}_i + c_7 * \text{fasting insulin}_I$$

$$(1)$$

wherein BMI1 is the subject's body mass index before the dietary intervention and BMI2 is the subject's predicted body mass index after the dietary intervention; and wherein c1, c2, c3, c4, c5, c6, and c7 are positive integers.

For example, the formula for BMI2 may be represented by formula (2):

$$\text{BMI2} = -1.25 + 0.35 \text{ (if subject is female)} + 0.9 \text{ (initial body mass index, BMI1)} + 0.003 \text{ (age in years)} - 0.2 \text{ (level of factor VII in units)} - 0.003 \text{ (level of fructosamine, micromole/L)} - 0.007 \text{ (level of adiponectin, microg/mL)} + 0.01 \text{ (level of fasting insulin, micromU/mL)}$$

$$(2)$$

According to a further aspect, the present invention provides a method for optimizing one or more dietary interventions for a subject comprising predicting the degree of weight loss attainable by the subject according to a method as defined herein, and applying the dietary intervention to the subject.

In a further aspect, the present invention provides a method for predicting the body mass index that a subject would be expected to attain from a dietary intervention (BMI2), wherein the method comprises determining the level of fructosamine, factor VII, adiponectin and insulin in one or more samples obtained from the subject, and predicting BMI2 using formula (1) or formula (2) as described hereinabove.

In a further aspect of the present invention there is provided a method for selecting a modification of lifestyle of a subject, the method comprising (a) performing a method as defined herein, and (b) selecting a suitable modification in lifestyle based upon the degree of weight loss predicted.

In one embodiment, the modification of lifestyle comprises a dietary intervention. The dietary intervention may comprise administering at least one diet product to the subject. For example, the dietary intervention may be a low calorie diet. A low calorie diet may comprise a decreased consumption of fat and/or an increase in consumption of low fat foods. By way of example only, low fat foods may include wholemeal flour and bread, porridge oats, high-fibre breakfast cereals, wholegrain rice and pasta, vegetables and fruit, dried beans and lentils, baked potatoes, dried fruit, walnuts, white fish, herring, mackerel, sardines, kippers, pilchards, salmon and lean white meat.

In a further aspect of the disclosure there is provided a diet product for use as part of a low calorie diet for weight loss, wherein the diet product is administered to a subject that is predicted to attain a degree of weight loss by the methods described herein.

In one aspect, the diet product may comprise a product such as Optifast® or Modifast®. This may be supplemented with three portions of non-starchy vegetables such that the total energy intake is about 2.5 MJ (600 kcal/day). This may be be further supplemented with at least 2 L of water or other energy free beverages per day.

In another aspect, the diet product may comprise, for example, a composition which is 46.4% carbohydrate, 32.5% protein and 20.1% with fat, vitamins, minerals and trace elements; 2.1MJ per day (510 kcal/day); This may be supplemented with three portions of non-starchy vegetables such that the total energy intake is about 2.5 MJ (600 kcal/day). This may be be further supplemented with at least 2 L of water or other energy free beverages per day.

In a further aspect of the disclosure there is provided a diet product for use in treating obesity or an obesity-related disorder, wherein the diet product is administered to a subject that is predicted to attain a degree of weight loss by the methods defined herein.

In a further aspect of the disclosure there is provided the use of a diet product in a low calorie diet for weight loss wherein the diet product is administered to a subject that is predicted to attain a degree of weight loss by the methods defined herein.

In a further aspect of the present invention, there is provided a computer program product comprising computer implementable instructions for causing a programmable computer to predict the degree of weight loss attainable by a subject according to the methods described herein.

In a further aspect of the disclosure there is provided a computer program product comprising computer implementable instructions for causing a programmable computer to predict the degree of weight loss given the levels of one or more biomarkers from the user, wherein the biomarkers are selected from fructosamine and factor VII. Preferably the biomarkers also include adiponectin and/or insulin.

In a further aspect of the present invention, there is provided a kit for predicting the degree of weight loss attainable by a subject following a dietary intervention, wherein said kit comprises an antibody specific for factor VII and an antibody specific for glycated albumin. In one embodiment, the kit further comprises an antibody specific for adiponectin and/or an antibody specific for insulin.

DETAILED DESCRIPTION OF THE INVENTION

**Predicting the degree of weight loss**

The present invention relates in one aspect to a method of predicting the degree of weight loss attainable by applying one or more dietary interventions to a subject. In particular embodiments, the method may be used to make an informed prediction of the subject's capacity to lose weight, and select or adjust one or more dietary intervention accordingly. For example, where the dietary intervention is a low calorie diet, the method could be used to select the appropriate diet for the subject or to adjust the daily calorie intake or duration of a particular diet to affect the degree of weight loss, or to increase compliance to the low calorie diet by setting realistic expectations for the subject. The method may also be used to assist in modifying the lifestyle of a subject.

The method provides a skilled person with a useful tool for assessing which subjects will most likely benefit from a particular dietary intervention, e.g. a low calorie diet. The present method therefore enables dietary interventions such as a low calorie diet and modifications in lifestyle to be optimised.

Weight loss as defined herein may refer to a reduction in parameters such as weight (e.g. in kilograms), body mass index ($kgm^{-2}$), or waist circumference (e.g. in centimetres), or waist-hip ratio (e.g. in centimetres). Weight loss may be calculated by subtracting the value of one or more of the aforementioned parameters at the end of the dietary intervention from the value of said parameter at the onset of the dietary intervention. Preferably, the degree of weight loss is represented by the body mass index that a subject is predicted to attain by applying the dietary intervention.

The degree of weight loss may be expressed as a percentage of a subject's body weight (e.g. in kilograms) or body mass index (kgm$^{-2}$). For example, a subject may be predicted to lose at least 10% of their initial body weight, at least 8% of their initial body weight, or at least 5% of their initial body weight. By way of example only, a subject may be predicted to lose between 5 and 10 % of their initial body weight.

In one embodiment, the percentage may be associated with an obesity-related disorder. For example, a degree of weight loss of at least 10% of initial body weight results in a considerable decrease in risk for obesity related co-morbidities.

Based on the degree of weight loss predicted using the methods defined herein, subjects may be stratified into one or more groups or categories. For example, subjects may be stratified according to whether or not they are predicted to lose a significant amount of weight.

## Subject

Preferably the subject is a mammal, preferably a human. The subject may alternatively be a non-human mammal, including for example, a horse, cow, sheep or pig. In one embodiment, the subject is a companion animal such as a dog or a cat.

## Sample

The present invention comprises a step of determining the level of one or more biomarkers in one or more samples obtained from a subject.

Preferably the sample is derived from blood or urine. More preferably the sample is derived from blood. The sample may contain a blood fraction or may be wholly blood. The sample preferably comprises blood plasma or serum, most preferably blood plasma. Techniques for collecting samples from a subject are well known in the art.

## Dietary Intervention

By the term "dietary intervention" is meant an external factor applied to a subject which causes a change in the subject's diet. In one embodiment, the dietary intervention is a low calorie diet.

Preferably the low calorie diet comprises a calorie intake of about 600 to about 1500 kcal/day, more preferably about 600 to about 1200 kcal/day, most preferably about 800 kcal/day. In one embodiment, the low calorie diet may comprise a predetermined amount (in grams) of vegetables per day. Preferably up to about 400g vegetables/day, e.g. about 200g vegetables/day.

The low calorie diet may comprise administration of at least one diet product. The diet product may be a meal replacement product or a supplement product which may e.g. suppress the subject's appetite. The diet product can include food products, drinks, pet food products, food supplements, nutraceuticals, food additives or nutritional formulas.

In one embodiment, the diet may comprise a product such as Optifast® or Modifast®. This may be supplemented with three portions of non-starchy vegetables such that the total energy intake is about 2.5 MJ (600 kcal/day). This may be be further supplemented with at least 2 L of water or other energy free beverages per day.

In another embodiment, the diet may comprise, for example, a composition which is 46.4% carbohydrate, 32.5% protein and 20.1% with fat, vitamins, minerals and trace elements; 2.1MJ per day (510 kcal/day); This may be supplemented with three portions of non-starchy vegetables such that the total energy intake is about 2.5 MJ (600 kcal/day). This may be be further supplemented with at least 2 L of water or other energy free beverages per day.

In one embodiment, the low calorie diet has a duration of up to 12 weeks. Preferably the low calorie diet has a duration of between 6 and 12 weeks, preferably between 8 and 10 weeks, e.g. 8 weeks.

## Determining the level of one or more biomarkers in the sample

In one embodiment, the level of one or more biomarkers is determined prior to the dietary intervention. In another embodiment, the level of one or more biomarkers is determined prior to, and after the dietary intervention. The biomarker level may also be determined at predetermined times throughout the dietary intervention. These predetermined times may be periodic throughout the dietary intervention, e.g. every day or three days, or may depend on the subject being tested, the type of sample being analysed and/or the degree of weight loss which is predicted to be attained.

When obtained prior to the dietary intervention, the biomarker level may be termed the "fasting level". When obtained after the dietary intervention, the biomarker level may be termed the "calorie intake level". For example, the biomarker level may be determined at fasting, or at fasting and after calorie intake. Most preferably the fasting level of each biomarker is determined.

The level of the individual biomarker species in the sample may be measured or determined by any suitable method known in the art. For example, mass spectroscopy (MS) or antibody detection methods, e.g. enzyme-linked immunoabsorbent assay (ELISA) may be used. Other spectroscopic methods, chromatographic methods, labelling techniques, or quantitative chemical methods may also be used.

In one embodiment, the level of one or more biomarkers may be determined by staining the sample with a reagent that labels one or more of the biomarkers. "Staining" is typically a histological method which renders the biomarker detectable by microscopic techniques such as those using visible or fluorescent light. Preferably the biomarker is detected in the sample by immunohistochemistry (IHC). In IHC, the biomarker may be detected by an antibody which binds specifically to one or more of the biomarkers. Suitable antibodies are known or may be generated using known techniques. Suitable test methods for detecting antibody levels include, but are not limited to, an immunoassay such as an enzyme-linked immunosorbent assay, radioimmunoassay, Western blotting and immunoprecipitation.

The antibody may be a monoclonal antibody, polyclonal antibody, multispecific antibody (e.g., bispecific antibody), or fragment thereof provided that it specifically binds to the biomarker being detected. Antibodies may be obtained by standard techniques comprising immunizing an animal with a target antigen and isolating the antibody from serum. Monoclonal antibodies may be made by the hybridoma method first described by Kohler et al., Nature 256:495 (1975), or may be made by recombinant DNA methods (see, e.g., U.S. Pat. No. 4,816,567). The monoclonal antibodies may also be isolated from phage antibody libraries using the techniques described in Clackson et al., Nature 352:624-628 (1991) and Marks et al., J. Mol. Biol. 222:581-597 (1991), for example. The antibody may also be a chimeric or humanized antibody. Antibodies are discussed further below.

Two general methods of IHC are available; direct and indirect assays. According to the first assay, binding of antibody to the target antigen is determined directly. This direct assay uses a labelled reagent, such as a fluorescent tag or an enzyme-labelled primary antibody, which can be visualized without further antibody interaction.

In a typical indirect assay, unconjugated primary antibody binds to the antigen and then a labelled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for IHC may be labelled with a detectable moiety. Numerous labels are available, including radioisotopes, colloidal gold particles, fluorescent labels and various enzyme-substrate labels. Fluorescent labels include, but are not limited to, rare earth chelates (europium chelates), Texas Red, rhodamine, fluorescein, dansyl, Lissamine, umbelliferone, phycocrytherin and phycocyanin, and/or derivatives of any one or more of the above. The fluorescent labels can be conjugated to the antibody using known techniques.

Various enzyme-substrate labels are available, e.g. as disclosed in US 4,275,149. The enzyme generally catalyses a chemical alteration of the chromogenic substrate that can be detected microscopically, e.g. under visible light. For example, the enzyme may catalyse a colour change in a substrate, or may alter the fluorescence or chemiluminescence of the substrate. Examples of enzymatic labels include luciferases (e.g. firefly luciferase and bacterial luciferase; US 4,737,456), luciferin, 2,3-dihydrophthalazinediones, malate dehydrogenase, urease, peroxidase such as horseradish peroxidase (HRPO), alkaline phosphatase, beta-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are well known.

Typically the method comprises a step of detecting stained regions within the image. Pixels in the image corresponding to staining associated with the biomarker may be identified by colour transformation methods, for instance as disclosed in US 6,553,135 and US 6,404,916. In such methods, stained objects of interest may be identified by recognising the distinctive colour associated with the stain. The method may comprise transforming pixels of the image to a different colour space, and applying a threshold value to suppress background staining. For instance, a ratio of two of the RGB signal values may be formed to provide a means for discriminating colour information. A particular stain may be discriminated from background by the presence of a minimum value for a particular signal ratio. For instance pixels corresponding to a predominantly red stain may be identified by a ratio of red divided by blue (R/B) which is greater than a minimum value.

Kong et al., Am J Clin Nutr, 2013 Dec; 98(6):1385-94 describes the use of the avidin-biotin-peroxidase method and two independent investigators counting the number of positively stained cells.

In one embodiment, the biomarker level is compared with a reference value. In which case, the biomarker level in the sample and the reference value are determined using the same analytical method.

*Fructosamine*

Fructosamine is a compound that results from glycation reactions between a sugar and a primary amine. Biologically, fructosamines are recognised by fructosamine-3-kinase.

It is known in the art that fructosamine testing typically calculates the fraction of total serum proteins in a blood sample that have undergone glycation. Since albumin is the most common protein in blood, fructosamine levels typically reflect albumin glycation. Preferably the determination of the level of fructosamine in the present method involves measuring glycated albumin. Glycated albumin measurement typically involves calculating the glycated albumin peak area to the total albumin peak area, either as a ratio or a percentage. The skilled person will, however, be aware of other methods in the art for determining the level of fructosamine in a sample and these are also suitable for the present method. Such methods include the phenylhydrazine procedure, the furosine procedure, affinity chromatography, the 2-thiobarbituric acid colorimetric procedure and the nitroblue tetrazolium colorimetric procedure (Armbruster DA, Clin Chem 33:2153, 1987). The level of fructosamine in a sample is preferably measured in moles per litre (mol/L).

*Factor VII*

Factor VII is a blood-clotting protein. It is also known in the art as anti-hemophilic factor (AHF).

Methods for measuring the level of factor VII in a sample are known in the art. Cugno et al., Intern Emerg Med (2012) 7:237-242 for example describes the use of a commercially available one-stage prothrombin time-based assay obtained from Instrumentation Laboratory Company, Lexington, MA, USA. Lijnen, H.R. et al., Thrombosis Research 129 (2012) 74-79 describes the use of a Dade Behring BCSXP system (Siemens Healthcare Diagnostics, Deerfield IL). The level of factor VII in a sample is typically measured in arbitrary units.

*Adiponectin*

Adiponectin is a protein which is encoded in humans by the *ADIPOQ* gene. It is also referred to in the art as GBP-28, apM1, AdipoQ and Acrp30.

Methods for determining the level of adiponectin in a sample are known in the art. Kong et al., Am J Clin Nutr, 2013 Dec; 98(6):1385-94 and Lijnen, H.R. et al., Thrombosis Research 129 (2012) 74-79 both describe the use of an ELISA kit (R&D Systems Europe, Lille, France). Lijnen H.R. et al. describes how the adiponectin levels are measured using commercial ELISA's and plasminogen activator inhibitor-1 (PAI01) antigen.

The level of adiponectin is preferably measured in grams per millilitre (g/ml).

*Insulin*

Insulin is a peptide hormone produced by beta cells of the pancreas.

The level of insulin in a sample is preferably measured in international units per millilitre (IU/ml). The international unit is a unit of measurement for the amount of a substance; the mass or volume that constitutes one international unit varies based on which substance is measured. For insulin, 1 IU is equivalent to 0.0347 mg of human insulin (28.8 IU/mg). The international unit (IU) is sometimes abbreviated to U.

**Combinations of biomarkers**

Whilst individual biomarkers may have predictive value in the methods of the present invention, the quality and/or the predictive power of the methods may be improved by combining values from multiple biomarkers.

Thus the method of the present invention may involve determining the level of at least two biomarkers from those defined herein. For instance, the method may comprise determining the level of fructosamine and factor VII, fructosamine and adiponectin, fructosamine and insulin, fructosamine, factor VII and adiponectin, fructosamine, factor VII and insulin, or fructosamine, factor VII, adiponectin and insulin.

A method comprising detecting a combination of biomarkers including fructosamine, factor VII, adiponectin and insulin is particularly preferred.

In a particularly preferred embodiment, the method comprises determining the level of each of fructosamine, factor VII, adiponectin and insulin, where decreased levels of fructosamine and insulin and increased levels of factor VII and adiponectin in the sample is indicative of a greater degree of weight loss in the subject.

**Comparison to a reference or control**

The present method may further comprise a step of comparing the level of the individual biomarkers in the test sample to one or more reference or control values. The reference value may be associated with a pre-defined ability of a subject to lose weight following dietary intervention. In some embodiments, the reference value is a value obtained previously for a subject or group of subjects following a certain dietary intervention. The reference value may be based on an average level, e.g. a mean or median level, from a group of subjects following the dietary intervention.

**Combining the biomarker levels with anthropometric measures and/or lifestyle characteristics**

In one embodiment, the present method further comprises combining the level of the one or more biomarkers with one or more anthropometric measures and/or lifestyle characteristics of the subject. By combining this information, an improved predictive model is provided for the degree of weight loss attainable by a subject.

As is known in the art, an anthropometric measure is a measurement of a subject. In one embodiment, the anthropometric measure is selected from the group consisting of gender, age (in years), weight (in kilograms), height (in centimetres), and body mass index (in kg/m$^{-2}$). Other anthropometric measures will also be known to the skilled person in the art.

By the term "lifestyle characteristic" is meant any lifestyle choice made by a subject, this includes all dietary intake data, activity measures or data from questionnaires of lifestyle, motivation or preferences. In one embodiment, the lifestyle characteristic is whether the subject is a smoker or a non-smoker. This is also referred to herein as the smoking status of the subject.

In a preferred embodiment, levels of fructosamine, adiponectin, insulin and factor VII are determined for a sample from the subject and these levels are combined with the gender, age, smoking status and body mass index of the subject in order to predict the weight loss attainable by the subject. Preferably the degree of weight loss is represented by the body mass index that a subject is predicted to attain by applying the dietary intervention.

In one embodiment, the predicted body mass index (BMI2) is generally represented by formula (1):

$$\text{bmi2 } i = c1*\text{bmi1} i + c2 \text{ (if subject } i \text{ is female)} + c3* \text{ age} i - c4* \text{ factor VII } i +$$

$$c5*\text{fructosamine } i - c6*\text{adiponectin } i + c7*\text{fasting insulin } i$$

wherein BMI1 is the subject's body mass index before the dietary intervention and BMI2 is the subject's predicted body mass index after the dietary intervention; and wherein c1, c2, c3, c4, c5, c6, and c7 are positive integers.

The values of c1 to c7 typically depend on 1) the measurement units of all the variables in the model; and 2) provenance (ethnic background) of the considered subject. Each of the coefficients c1 to c7 can be readily determined for particular subject cohorts. As would be understood by the skilled person, a dietary intervention, for example a low calorie diet, may be applied to a subject cohort of interest, the levels of the biomarkers as defined herein may be determined and routine statistical methods may then be used in order to arrive at the values of c1 to c7. Such routine statistical methods may include multiple linear regression with calibration by bootstrap. It is possible to obtain the same estimates with generalized linear or additive models or any other regression-related model with various estimation algorithms, for example, elastic net, lasso, Bayesian approach etc. In a particularly preferred embodiment, the predicted body mass index (BMI2) is calculated by formula (2):

$$\text{BMI2} = -1.27 + 0.5 \text{ (if subject is female)} + 0.9 \text{ (initial body mass index, BMI1)} + 0.001$$

$$\text{(age in years)} - 0.014 \text{ (if subject is a non-smoker)} + 0.03 \text{ (level of factor VII in}$$

$$\text{units)} - 0.0004 \text{ (level of fructosamine, } \mu\text{mol/L)} - 0.002 \text{ (level of adiponectin,}$$

$$\mu\text{g/mL)} + 0.002 \text{ (level of fasting insulin, nU/mL)}$$

In one embodiment, the subject is European.

**Subject stratification**

The degree of weight loss predicted by the method of the present invention may also be compared to one or more pre-determined thresholds. Using such thresholds, subjects may be stratified into categories which are indicative of the degree of predicted weight loss, e.g. low, medium, high and/or very high predicted degree of weight loss. The extent of the divergence from the thresholds is useful to determine which subjects would benefit most from certain interventions. In this way, dietary intervention and modification of lifestyle can be optimised, and realistic expectations of the weight loss to be achieved by the subject can be set.

In one embodiment, the categories include weight loss resistant subjects and weight loss sensitive subjects.

By the term "weight loss resistant" is meant a predicted degree of weight loss which is less than a predetermined value. Preferably "weight loss resistant" is defined as a subject having a weight loss percentage inferior to a predetermined value e.g. a subject predicted to lose less weight than the 10th, 15th, 20th or 30th percentile of the expected weight loss for the subject.

Preferably the degree of weight loss is represented by the number of BMI units lost, where BMI loss = ((BMI1 -BMI2)*100)/BMI1, wherein BMI1 is the body mass index of the subject before the dietary intervention and BMI2 is the predicted body mass index of the subject after the dietary intervention.

By the term "weight loss sensitive" is meant a predicted degree of weight loss of more than a predetermined value. Preferably "weight loss sensitive" is defined as a subject having a weight loss percentage superior to a predetermined threshold value. For example a subject predicted to lose more weight than the 85th, 80th or 75th percentile of the expected weight loss.

The "expected weight loss" can be obtained from data of a population of subjects that have undergone the same dietary intervention as the one being tested.

In another embodiment, subjects may be stratified into categories "weight loss sensitive" or "weight loss resistant" which are indicative of the risk reduction of the subject for obesity or obesity-related disorders, e.g. low, medium, high and/or very high risk reduction. Low, medium and high risk reduction groups may be defined in terms of absolute weight loss, where the absolute weight loss relates to clinical criteria for obesity or a particular obesity-related disorder.

For example, if the aim is to reduce the risk for type 2 diabetes in an obese individual, "very high risk reduction" may be defined as those predicted to lose at least 10% body weight after the dietary intervention. This is in accordance with the criteria set out in Part II of the World Health Organ Tech Rep Ser. 2000;894:i-xii, 1-253). Moreover every 1% reduction in body weight of an obese person leads to a fall in systolic and diastolic blood pressure, and fall in low-density lipoprotein cholesterol, hence reduces the risk of cardio-vascular disease and dyslipidaemia respectively.

**Method for selecting a modification of lifestyle of a subject**

In a further aspect, the present invention provides a method for modifying the lifestyle of a subject. The modification in lifestyle in the subject may be any change as described herein, e.g. a change in diet, more exercise, a different working and/or living environment etc.

Preferably the modification is a dietary intervention as described herein. More preferably the dietary intervention includes the administration of at least one diet product. The diet product preferably has not previously been consumed or was consumed in different amounts by the subject. The diet product may be as described herein. Modifying a lifestyle of the subject also includes indicating a need for the subject to change his/her lifestyle, e.g. prescribing more exercise or stopping smoking.

For example, if a subject is not predicted to lose weight on a low calorie diet, a modification may include more exercise in the subject's lifestyle.

**Use of Diet Products**

In one aspect, the disclosure provides a diet product for use as part of a low calorie diet for weight loss. The diet product being administered to a subject that is predicted to attain a degree of weight loss by the methods described herein.

In another aspect, the disclosure provides a diet product for use in treating obesity or an obesity-related disorder, wherein the diet product is administered to a subject that is predicted to attain a degree of weight loss by the methods described herein.

The obesity-related disorder may be selected from the group consisting of diabetes (e.g. type 2 diabetes), stroke, high cholesterol, cardiovascular disease, insulin resistance, coronary heart disease, metabolic syndrome, hypertension and fatty liver. In a further aspect, the present invention provides the use of a diet product in a low calorie diet for weight loss where the diet product is administered to a subject that is predicted to attain a degree of weight loss by the methods described herein.

**Kits**

In a further aspect, the present invention provides a kit for predicting the degree of weight loss attainable by applying one or more dietary interventions to the subject.

The kit comprises an antibody specific for factor VII or an antibody specific for glycated albumin. The kit may also comprise an antibody specific for insulin and/or an antibody specific for adiponectin. Preferably the kit comprises an antibody specific for factor VII, an antibody specific for glycated albumin, an antibody specific for insulin and an antibody specific for adiponectin

The term antibody includes antibody fragments. Such fragments include fragments of whole antibodies which retain their binding activity for a target substance, Fv, F(ab') and F(ab')$_2$ fragments, as well as single chain antibodies (scFv), fusion proteins and other synthetic proteins which comprise the antigen-binding site of the antibody. Furthermore, the antibodies and fragments thereof may be humanised antibodies. The skilled person will be aware of methods in the art to produce the antibodies required for the present kit.

**Computer Program Product**

The methods described herein may be implemented as a computer program running on general purpose hardware, such as one or more computer processors. In some embodiments, the functionality described herein may be implemented by a device such as a smartphone, a tablet terminal or a personal computer.

In one aspect, the present invention provides a computer program product comprising computer implementable instructions for causing a programmable computer to predict the degree of weight loss based on the levels of biomarkers as described herein.

In another aspect, the disclosure provides a computer program product comprising computer implementable instructions for causing a device to predict the degree of weight loss given the levels of one or more biomarkers from the user, wherein the biomarkers are selected from fructosamine, factor VII or mixtures thereof. The biomarker levels may further include the adiponectin and/or insulin levels. Preferably the biomarker levels are fasting levels. The computer program product may also be given anthropometric measures and/or lifestyle characteristics from the user. As described herein, anthropometric measures include age, weight, height, gender and body mass index and lifestyle characteristics include smoking status.

In a particularly preferred embodiment, the user inputs into the device levels of fructosamine, adiponectin, factor VII and insulin, optionally along with age, body mass index, gender and smoking status. The device then processes this information and provides a prediction on the degree of weight loss attainable by the user from a dietary intervention.

The device may generally be a server on a network. However, any device may be used as long as it can process biomarker data and/or anthropometric and lifestyle data using a processor, a central processing unit (CPU) or the like. The device may, for example, be a smartphone, a tablet terminal or a personal computer and output information indicating the degree of weight loss attainable by the user.

Those skilled in the art will understand that they can freely combine all features of the present invention described herein, without departing from the scope of the invention as disclosed. Various preferred features and embodiments of the present invention will now be described by way of non-limiting examples.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992 and periodic supplements, Current Protocols in Immunology, John Wiley & Sons, New York, NY.

EXAMPLES

**Example 1 - Predicting degree of weight loss after low calorie diet**

Subjects were participants in the Diogenes study. This study is a pan-European, randomised and controlled dietary intervention study investigating the effects of dietary protein and glycaemic index on weight loss and weight maintenance in obese and overweight families in eight European centres (Larsen et al., Obesity reviews (2009), 11, 76-91).

Example 1 involved 938 European individuals of which 782 finished the 8 week LCD program and 714 had all the required measurements with ranges admissible for a living subject. General parameters for the individuals are shown in Table 1.

**Table 1: General characteristics of individuals who followed the low calorie diet**

| Parameter | Average (standard deviation) |
|---|---|
| women percentage | 64 (not applicable) |

(continued)

| Parameter | Average (standard deviation) |
|---|---|
| age | 41.5 (6.3) |
| BMI before LCD (BMI1) | 34.6 (4.9) |
| BMI after LCD (BMI2) | 30.8 (4.4) |
| fructosamin fasting level (micromol/L) | 207.8 (24.1) |
| insulin fasting level (microIU/mL) | 10.9 (6.1) |
| factor VII fasting level (arbitrary units) | 1.08 (0.2) |
| adiponectin level (microg/mL) | 9.0 (4.4) |

Blood samples were taken before and after the completion of the 8 week LCD periods and plasma levels of fructosamine, adiponectin, factor VII and insulin were determined. It was found that fasting levels of these biomarkers determined before the LCD intervention is associated with an individual's capacity to lose weight.

Multiple anthropometric measures were also taken prior to the dietary intervention, including age, weight and height (from which the BMI - body mass index - was derived as weight/height$^2$) and gender. For technical reasons some biomarker measurements failed for 62 subjects so the data available is for the remaining 714 subjects. These anthropometric measures were conducted using standard clinical practices.

All the variables measured prior to the dietary intervention were evaluated for being separately and jointly predictors of BMI2 given BMI1. Multiple statistical models were evaluated using available tools known in the art such as, for example, generalized additive and linear models with and without interactions with Gaussian or Gamma distributed outcome) (R software) and retained the following predictive model (formula (2)) based on the prediction quality using cross-validation- bootstrap of the multiple linear regression model and its coefficients):

$$BMI2 = -1.27 + 0.9*BMI1_i + 0.5 \text{ (if subject i is female)} + 0.001*age - 0.014 \text{ (if non-smoking)} +$$

$$0.03*factor\ VII_i - 0.0004*fructosamine_i - 0.002*adiponectin_i + 0.002*fasting\ insulin_i$$

$$(2)$$

The overall prediction accuracy of the model in this study was determined to be 96% of the total variation (adjusted $R^2 = 0.96$). The predicted BMI2 and the levels of each of the biomarkers is shown in Table 2.

**Table 2: Example of predicted BMI2 with 95% confidence interval and observed BMI2**

| Gender | Fructosamine | Insulin | Factor VII | Adiponectin | BMI1 | Predicted BMI2 (95% confidence) | Observed BMI2 |
|---|---|---|---|---|---|---|---|
| Female | 219 | 8.68 | 1.50 | 5.70 | 29.3 | 25.6 (23.6, 27.6) | 25.5 |
| Male | 201 | 18.3 | 1.59 | 4.95 | 39.1 | 35.1 (33.1, 37.1) | 35.6 |
| Female | 193 | 15.9 | 1.17 | 7.32 | 35.9 | 32.1 (30.1, 34.1) | 30.6 |
| Male | 220 | 4.19 | 1.06 | 14.10 | 28.1 | 23.9 (21.9, 25.9) | 24.8 |

Table 3 contains the p-values of all of the coefficients of the predictive model for the average expected BMI2 (using bootstrapped estimate distributions for regression model).

**Table 3: Coefficient estimate signs and p-values computed using bootstrapped estimates of the predictive model (when predicting average expected bmi2).**

| | coefficient | p-value based on the bootstrapped estimates |
|---|---|---|
| Factor VII | c4 | < 0.08 |
| Fructosamine | c5 | < 0.03 |
| Adiponectin | c6 | < 0.25 |
| Insulin | c7 | < 0 12 |

**Example 2 - Stratification of subjects according to predicted weight loss**

Example 2 involves the same subjects as the example 1 though instead of predicting the BMI2 (BMI after the low calorie intervention) we focus on predicting the probability of a subject to be a "weight loss sensitive" or "weight loss resistant".

Table 4 contains biomarkers' coefficients with respective significance for predicting the probability of being "weight loss resistant" and "weight loss sensitive", where the probability is adjusted for age and gender.

**Table 4: Biomarkers coefficients signs and p-values in prediction of probability of being "weight loss resistant" and "weight loss sensitive" (adjusted for age and gender) with following definitions and cutoffs: "weight loss resistance" means predicted to lose less BMI than the 30th (15th) percentile of the expected bmi loss; "weight loss sensitive" means predicted to lose more BMI than the 70th (85th) percentile of the expected bmi loss. Only correlations with p-values smaller than 0.1 are reported.**

| | Coefficient in prediction of probability of being "weight loss resistant" (p-value) | Coefficient in prediction of probability of being "weight loss sensitive" (p-value) |
|---|---|---|
| 15th and 85th percentile cutoffs of BMI loss | | |
| Factor VII | Negative (p-val<0.01) | |
| Fructosamine | Positive (p-val< 0.1) | |
| 30th and 70th percentile cutoffs of BMI loss | | |
| Fructosamine | Positive (p-val< 0.004) | |
| Adiponectin | Negative (p-val<0.04) | Positive (p-val<0.07) |
| Insulin | | Negative (p-val<0.01) |

**Claims**

1. A method for predicting the degree of weight loss attainable by applying one or more dietary interventions to a subject, said method comprising:
   determining the level of fructosamine in one or more samples obtained from the subject.

2. The method according to claim 1, wherein the method further comprises determining the level of factor VII in one or more samples.

3. The method according to claim 1 or claim 2, wherein the method further comprises determining the level of adiponectin in one or more samples.

4. The method according to claims 1 to 3, wherein the method further comprises determining the level of insulin in one or more samples.

5. The method according to any one of claims 1 to 4, wherein levels of each of fructosamine, factor VII, adiponectin and insulin are determined, and decreased levels of fructosamine and insulin and increased levels of factor VII and adiponectin in the sample is indicative of a greater degree of weight loss in the subject.

6. The method according to any one of claims 1 to 5, wherein the one or more samples are derived from blood.

7. The method according to anyone of claims 1 to 6, wherein the fructosamine levels are determined by measuring glycated albumin.

8. A method for optimizing one or more dietary interventions for a subject comprising:

predicting the degree of weight loss attainable by the subject according to a method as defined in any one of claims 1 to 7; and
applying the dietary intervention to the subject.

9. A method for predicting the body mass index that a subject would be expected to attain from a dietary intervention (BMI2), wherein the method comprises:

a. determining the level of fructosamine, Factor VII, adiponectin and insulin in one or more samples obtained from the subject; and
b. predicting BMI2 using formula (1):

$$\text{bmi2 i} = c1*\text{bmi1i} + c2 \text{ (if subject i is female)} + c3* \text{agei} - c4* \text{ factor VII i} +$$

$$c5*\text{fructosamine i}$$

$$- c6*\text{adiponectin i} + c7*\text{fasting insulin i}$$

$$(1)$$

wherein BMI1 is the subject's body mass index before the dietary intervention and BMI2 is the subject's predicted body mass index after the dietary intervention; and
wherein c1, c2, c3, c4, c5, c6, and c7 are positive integers.

10. The method according to claim 9, wherein BMI2 is predicted using formula (2):

$$\text{BMI2} = -1.25 + 0.35 \text{ (if subject is female)} + 0.9 \text{ (initial body mass index, BMI1)} +$$

$$0.003 \text{ (age in years)} - 0.02 \text{ (level of factor VII in units)} -$$

$$0.003 \text{ (level of fructosamine, micromole/L)} -$$

$$0.007 \text{ (level of adiponectin, microg/mL)} +$$

$$0.01 \text{ (level of fasting insulin, micromU/mL)}$$

$$(2)$$

11. A method for selecting a modification of lifestyle of a subject, the method comprising:

a. performing a method as described in any of claims 1 to 10; and
b. selecting a suitable modification in lifestyle based upon the degree of weight loss predicted in step (a).

12. The method according to claim 11, wherein the modification of lifestyle in the subject comprises a dietary intervention.

13. A computer program product comprising computer implementable instructions for causing a programmable computer to perform the method of any of claims 1 to 12.

14. The product according to claim 13. wherein the computer program product is further given anthropometric measures and/or lifestyle characteristics from the user, wherein the anthropometric measures are selected from the group consisting of age, weight, height, gender and body mass index and the lifestyle characteristics is whether the subject is a smoker or a non-smoker.

15. A kit for predicting the degree of weight loss attainable by a subject following a dietary intervention, wherein said kit comprises:

a. an antibody specific for factor VII; and
b. an antibody specific for glycated albumin

**Patentansprüche**

1. Verfahren zum Vorhersagen des Grads an Gewichtsverlust, der durch Anwenden einer oder mehrerer diätetischer Interventionen auf eine Versuchsperson erreichbar ist, wobei das Verfahren Folgendes umfasst:
Bestimmen des Gehalts an Fruktosamin in einer oder mehreren von der Versuchsperson erhaltenen Proben.

2. Verfahren nach Anspruch 1, wobei das Verfahren ferner das Bestimmen des Gehalts an Faktor VII in einer oder mehreren Proben umfasst.

3. Verfahren nach Anspruch 1 oder 2, wobei das Verfahren ferner das Bestimmen des Gehalts an Adiponektin in einer oder mehreren Proben umfasst.

4. Verfahren nach Anspruch 1 bis 3, wobei das Verfahren ferner das Bestimmen des Gehalts an Insulin in einer oder mehreren Proben umfasst.

5. Verfahren nach einem der Ansprüche 1 bis 4, wobei jeweils der Gehalt an Fruktosamin, Faktor VII, Adiponektin und Insulin bestimmt wird und ein verringerter Gehalt an Fructosamin und Insulin und ein erhöhter Gehalt an Faktor VII und Adiponektin in der Probe auf einen höheren Grad an Gewichtsverlust in der Versuchsperson hindeutet.

6. Verfahren nach einem der Ansprüche 1 bis 5, wobei die eine oder mehreren Proben aus Blut stammen.

7. Verfahren nach einem der Ansprüche 1 bis 6, wobei der Gehalt an Fruktosamin jeweils durch Messen von glykosyliertem Albumin bestimmt wird.

8. Verfahren zum Optimieren eines oder mehrerer diätetischer Interventionen für eine Versuchsperson, umfassend:
Vorhersagen des Grads an Gewichtsverlust, der von der Versuchsperson erreichbar ist, gemäß einem Verfahren, das in einem der Ansprüche 1 bis 7 definiert ist; und
Anwenden der diätetischen Intervention auf die Versuchsperson.

9. Verfahren zum Vorhersagen des Body-Mass-Index, den eine Versuchsperson aufgrund einer diätetischen Intervention (BMI2) erwarten würde, wobei das Verfahren Folgendes umfasst:

a. Bestimmen des Gehalts an Fruktosamin, Faktor VII, Adiponektin und Insulin in einer oder mehreren von der Versuchsperson erhaltenen Proben; und
b. Vorhersagen des BMI2 anhand der Formel (1):

$$BMI2_i = c_1 * BMI1_i + c_2 \text{ (wenn Versuchsperson } i \text{ weiblich ist)} + c_3 * \text{Alter}_i - c_4 * \text{Faktor VII}_i +$$

$$c_5 * \text{Fruktosamin}_i$$

$$- c_6 * \text{Adiponektin}_i + c_7 * \text{Nüchterninsulin}_i$$

$$(1)$$

wobei BMI1 der Body-Mass-Index der Versuchsperson vor der diätetischen Intervention ist und BMI2 der vorhergesagte Body-Mass-Index der Versuchsperson nach der diätetischen Intervention ist; und wobei c1, c2, c3, c4, c5, c6 und c7 positive ganze Zahlen sind.

10. Verfahren nach Anspruch 9, wobei BMI2 anhand der Formel (2) vorhergesagt wird:

$$\text{BMI2} = -1{,}25 + 0{,}35 \text{ (wenn Versuchsperson weiblich ist)} + 0{,}9 \text{ (anfänglicher}$$
$$\text{Body-Mass-Index, BMI1)} +$$
$$0{,}003 \text{ (Alter in Jahren)} - 0{,}02 \text{ (Gehalt an Faktor VII in Einheiten)} -$$
$$0{,}003 \text{ (Gehalt an Fruktosamin, Mikromol/l)} -$$
$$0{,}007 \text{ (Gehalt an Adiponektin, Mikrogramm/ml)} +$$
$$0{,}01 \text{(Gehalt an Nüchterninsulin, MikromU/ml)}$$

$$(2)$$

11. Verfahren zum Auswählen einer Veränderung der Lebensweise einer Person, wobei das Verfahren Folgendes umfasst:

    a. Durchführen eines Verfahrens, wie in einem der Ansprüche 1 bis 10 beschrieben; und
    b. Auswählen einer geeigneten Veränderung der Lebensweise basierend auf dem in Schritt (a) vorhergesagten Grad des Gewichtsverlusts.

12. Verfahren nach Anspruch 11, wobei die Veränderung der Lebensweise bei der Versuchsperson eine diätetische Intervention umfasst.

13. Computerprogrammprodukt, umfassend computerimplementierbare Anweisungen, um einen programmierbaren Computer zu veranlassen, das Verfahren nach einem der Ansprüche 1 bis 12 auszuführen.

14. Produkt nach Anspruch 13, wobei in das Computerprogrammprodukt ferner anthropometrische Maße und/oder Merkmale der Lebensweise vom Benutzer eingegeben werden, wobei die anthropometrischen Maße aus der Gruppe ausgewählt werden, die aus Alter, Gewicht, Größe, Geschlecht und Body-Mass-Index besteht, und Merkmale der Lebensweise sind, ob die Versuchsperson Raucher oder Nichtraucher ist.

15. Kit zum Vorhersagen des Grads an Gewichtsverlust, der von einer Versuchsperson nach einer diätetischen Intervention erreicht werden kann, wobei das Kit Folgendes umfasst:

    a. einen Antikörper, der für Faktor VII spezifisch ist; und
    b. einen Antikörper, der für glykiertes Albumin spezifisch ist.

**Revendications**

1. Procédé pour prédire le degré de perte de poids pouvant être atteint par l'application d'une ou plusieurs interventions alimentaires à un sujet, ledit procédé comprenant :
la détermination du taux de fructosamine dans un ou plusieurs échantillons prélevés sur le sujet.

2. Procédé selon la revendication 1, dans lequel le procédé comprend en outre la détermination du taux de facteur VII dans un ou plusieurs échantillons.

3. Procédé selon la revendication 1 ou la revendication 2, dans lequel le procédé comprend en outre la détermination du taux d'adiponectine dans un ou plusieurs échantillons.

4. Procédé selon les revendications 1 à 3, dans lequel le procédé comprend en outre la détermination du taux d'insuline dans un ou plusieurs échantillons.

**5.** Procédé selon l'une quelconque des revendications 1 à 4, dans lequel les taux de chacun de la fructosamine, du facteur VII, de l'adiponectine et de l'insuline sont déterminés, et des taux réduits de fructosamine et d'insuline et des taux élevés de facteur VII et d'adiponectine dans l'échantillon sont indicatifs d'un degré plus élevé de perte de poids chez le sujet.

**6.** Procédé selon l'une quelconque des revendications 1 à 5, dans lequel les un ou plusieurs échantillons sont dérivés de sang.

**7.** Procédé selon l'une quelconque des revendications 1 à 6, dans lequel les taux de fructosamine sont déterminés en mesurant l'albumine glyquée.

**8.** Procédé d'optimisation d'une ou plusieurs interventions alimentaires pour un sujet comprenant : la prédiction du degré de perte de poids pouvant être atteint par le sujet selon un procédé tel que défini selon l'une quelconque des revendications 1 à 7 ; et
l'application de l'intervention alimentaire au sujet.

**9.** Procédé de prédiction de l'indice de masse corporelle qu'un sujet pourrait attendre d'une intervention alimentaire (BMI2), dans lequel le procédé comprend :

   a. la détermination du taux de fructosamine, de facteur VII, d'adiponectine et d'insuline dans un ou plusieurs échantillons prélevés sur le sujet ; et
   b. la prédiction du BMI2 à l'aide de la formule (1) :

$$bmi2\ i = c1*bmi1i + c2\ (\text{si le sujet } i \text{ est une femme}) + c3*âgei - c4*\text{facteur VII } i +$$
$$c5*\text{fructosamine } i$$

$$- c6*\text{adiponectine } i + c7*\text{insuline à jeun } i$$

$$(1)$$

   dans laquelle BMI1 est l'indice de masse corporelle du sujet avant l'intervention alimentaire et BMI2 est l'indice de masse corporelle prédit du sujet après l'intervention alimentaire ; et
   dans laquelle c1, c2, c3, c4, c5, c6, et c7 sont des nombres entiers positifs.

**10.** Procédé selon la revendication 9, dans lequel le BMI2 est prédit à l'aide de la formule (2) :

$$BMI2 = -1{,}25 + 0{,}35\ (\text{si le sujet est une femme}) + 0{,}9\ (\text{indice de masse corporelle}$$
$$\text{initial, BMI1}) +$$
$$0{,}003\ (\text{âge en années}) - 0{,}02\ (\text{taux de facteur VII en unités}) -$$
$$0{,}003\ (\text{taux de fructosamine, micromole/L}) -$$
$$0{,}007\ (\text{taux d'adiponectine, microg/mL}) +$$
$$0{,}01\ (\text{taux d'insuline à jeun, micromU/mL})$$

$$(2)$$

**11.** Procédé de sélection d'une modification du style de vie d'un sujet, le procédé comprenant :

   (a). l'application d'un procédé tel que décrit selon l'une quelconque des revendications 1 à 10 ; et
   b. la sélection d'une modification appropriée du style de vie sur la base du degré de perte de poids prédit à l'étape (a).

12. Procédé selon la revendication 11, dans lequel la modification du style de vie chez le sujet comprend une intervention alimentaire.

13. Produit programme informatique comprenant des instructions exécutables par ordinateur pour amener un ordinateur programmable à exécuter le procédé selon l'une quelconque des revendications 1 à 12.

14. Produit selon la revendication 13, dans lequel le produit programme informatique reçoit en outre des mesures anthropométriques et/ou des caractéristiques de style de vie de l'utilisateur, dans lequel les mesures anthropométriques sont choisies parmi le groupe constitué de l'âge, du poids, de la taille, du sexe et de l'indice de masse corporelle et les caractéristiques de style de vie indiquent si le sujet est fumeur ou non-fumeur.

15. Kit pour prédire le degré de perte de poids pouvant être atteint chez un sujet après une intervention alimentaire, dans lequel ledit kit comprend :

  a. un anticorps spécifique pour le facteur VII ; et
  b. un anticorps spécifique pour l'albumine glyquée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110124121 A
- EP 2420843 A
- US 4816567 A
- US 4275149 A
- US 4737456 A
- US 6553135 B
- US 6404916 B

### Non-patent literature cited in the description

- *World Health Organ Tech Rep Ser.,* 2000, vol. 894 (i-xii), 1-253
- **GHOSH, S. et al.** *Obesity (Silver Spring),* 2011, vol. 19 (2), 457-463
- **LIJNEN et al.** *Thromb Res.,* 12 January 2012, vol. 9 (1), 74-9
- **CUGNO et al.** *Intern Emerg Med.,* June 2012, vol. 7 (3), 237-42
- **BLADBJERG et al.** *Br J Nutr.,* December 2010, vol. 104 (12), 1824-30
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **KONG et al.** *Am J Clin Nutr,* December 2013, vol. 98 (6), 1385-94
- **ARMBRUSTER DA.** *Clin Chem,* 1987, vol. 33, 2153
- **CUGNO et al.** *Intern Emerg Med,* 2012, vol. 7, 237-242
- **LIJNEN, H.R. et al.** *Thrombosis Research,* 2012, vol. 129, 74-79
- **J. SAMBROOK ; E. F. FRITSCH ; T. MANIATIS.** Molecular Cloning: A Laboratory Manual. Cold Spring Harbor Laboratory Press, 1989, 1-3
- Current Protocols in Molecular Biology. John Wiley & Sons
- **B. ROE ; J. CRABTREE ; A. KAHN.** DNA Isolation and Sequencing: Essential Techniques. John Wiley & Sons, 1996
- **J. M. POLAK ; JAMES O'D. MCGEE.** In Situ Hybridization: Principles and Practice. Oxford University Press, 1990
- Oligonucleotide Synthesis: A Practical Approach. Irl Press, 1984
- Methods of Enzymology: DNA Structure Part A: Synthesis and Physical Analysis of DNA. **D. M. J. LILLEY ; J. E. DAHLBERG.** Methods in Enzymology. Academic Press, 1992
- **E. M. SHEVACH ; W. STROBER.** Current Protocols in Immunology. John Wiley & Sons, 1992
- **LARSEN et al.** *Obesity reviews,* 2009, vol. 11, 76-91